**Europäisches Patentamt**

**European Patent Office**

**Office européen des brevets**

(19)

(11) Publication number: **0 181 497**

**B1**

(12) **EUROPEAN PATENT SPECIFICATION**

(45) Date of publication of patent specification: **21.03.90**

(51) Int. Cl.⁵: **C 02 F 1/28, C 22 B 3/00**

(21) Application number: **85112810.8**

(22) Date of filing: **09.10.85**

(54) **Metal recovery.**

(30) Priority: **17.10.84 US 661917**
**20.09.85 US 777061**

(43) Date of publication of application:
**21.05.86 Bulletin 86/21**

(45) Publication of the grant of the patent:
**21.03.90 Bulletin 90/12**

(84) Designated Contracting States:
**AT BE CH DE FR IT LI LU NL SE**

(56) References cited:
**DE-A-2 404 562**
**US-A-4 293 333**

**ENVIRON. BIOGEOCHEM. GEOMICROBIOL.,**
**PROC. INT. SYMP., 3rd 1977, pages 975-987,**
**edited by Krumbein, Wolfgang E. Ann Arbor**
**Sci., Mich., US; T.J. BEVERIDGE: "The**
**interaction of metals in aqueous solution with**
**bacterial cell walls from Bacillus Subtilis"**

(73) Proprietor: **Advanced Mineral Technologies, Inc.**
**1309 Lopezville Road P.O. Box 1339**
**Socorro New Mexico 87801 (US)**

(72) Inventor: **Brierley, James A.**
**1103 Bullock**
**Socorro New Mexico (US)**
Inventor: **Brierley, Corale L.**
**1103 Bullock**
**Socorro New Mexico (US)**
Inventor: **Decker, Raymond f.**
**701 E. Seventh**
**Houghton Michigan (US)**
Inventor: **Goyak, George M.**
**RD 1, Box 122**
**Harmony Pennsylvania (US)**

(74) Representative: **Greenstreet, Cyril Henry et al**
**Haseltine Lake & Co. Hazlitt House 28**
**Southampton Buildings Chancery Lane**
**London WC2A 1AT (GB)**

Courier Press, Leamington Spa, England.

## Description

The present invention is directed to the treatment of aqueous solutions containing cations of heavy metals with a biomass reaction product derived from a microorganism, such as *Bacillus subtilis*, selective to the removal of heavy metals from solution. The invention is also directed to a process of enhancing metal uptake properties of microorganisms and to relatively stable biomass products produced by said process having enhanced metal uptake properties.

### Background of the invention and the prior art

Waste waters from many industrial processes, such as metal finishing, which contain a variety of metal ions some of which are toxic and some of which are valuable, are generated in large quantities. These liquids cannot be released into streams or sewers without causing environmental damage. Furthermore, such release would, in many cases, represent a violation of stringent environmental regulations. Treatment of such waters to remove the metal ions is required. Existing processes for treating such waste waters suffer from many disadvantages, among which are included high cost, production of metal-containing sludges which are difficult to treat for metal recovery and hence are dumped in landfills, complex technology, etc. The use of caustic precipitation, sulfide precipitation, electrolysis, evaporation, reverse osmosis, ion exchange, etc., are all known and suffer form one or more difficulty in terms of energy requirement, limited applicability, low absorption capacity, requirement for precise control, etc.

Attempts to use biomass of living microbes for metal recovery are reported in US—A—4407954 and 4293333. T. J. Beveridge, et al. have reported that cell walls of *B. subtilis* will take up metals from solution (J. Bacteriol, 1976, 127(3), 1502—18). However, living microbes must be cultured, an expensive, capital-intensive process, and are subject to contamination by other microbes and inhibition by high metal concentrations or extremes of pH and other toxic components of waste streams. They are also subject to putrefaction. US—A—4293334 and 4320093 disclose use of nonliving biomass derived from microbes but these processes display limited metal sorption or uptake capacity. DE—A—2 404 562 discloses an absorbant which comprises heat-treated microbial cells which are capable of selectively absorbing heavy metals, from waste-water.

### Brief description of the invention

One embodiment of the invention contemplates the recovery of the metal present in cationic form in aqueous streams by using a relatively insoluble solid biomass product in particulate form derived chemically from microorganisms characterized by cell wall structures, a preferred microorganism being the bacterium, *Bacillus subtilis*. The sorption or metal uptake capacity for metal ions is dramatically improved by treating the microorganism with a caustic solution which converts it to a substantially solid biomass reaction product. The term "solid" also includes a reaction product having a porous structure.

The term "caustic" is meant to cover alkaline solutions having a relatively high intrinsic pH, e.g., in excess of 9, preferably in excess of 10 or 11, prior to reaction with the microorganism, the alkaline solution being one which when reacted with a selected microorganism produces a relatively solid biomass reaction product having enhanced metal uptake capacity compared to the metal uptake properties of the microorganism prior to the caustic treatment. The term caustic is defined in Webster's Collegiate Dictionary as capable of destroying or eating away by chemical reaction, such as occurs during caustic treatment of microorganisms in accordance with the invention.

The term "sorption" is used in the broad sense to include all forms of metal uptake, whether by adsorption, absorption, ionic bonding, among other forms of metal uptake.

Another embodiment of the invention resides in a process for enhancing the metal uptake properties of microorganisms and the biomass reaction produced by the process. The microorganisms selected are those which have cell wall structures (e.g., *Bacillus subtilis*) which when reacted with a caustic solution form a substantially solid biomass reaction product having enhanced metal uptake properties. The "causticized" biomass or reaction product having the enhanced uptake capacity is slightly alkaline when dispersed in water. Thus, the invention provides a causticized microorganism in the form of a substantially solid biomass reaction product, the product being preferably in the particulate form.

In producing the solid biomass reaction product, solutions of NaOH and KOH are preferred having concentrations ranging from about 0.25 molar to 1.25 molar. The microorganism, e.g., *Bacillus subtilis*, is treated with the caustic solution at temperatures ranging up to boiling, preferably above ambient temperature, e.g., from about 50°C to 100°C, to form the biomass reaction product which is then washed to remove excess alkaline solution and then dried. The treated dried biomass may be in the form of hard, grindable bodies, such as plates. The biomass reaction product in the particulate form is slightly alkaline and can be used in a column to contact the metal-containing aqueous stream.

When the metal-containing aqueous stream is contacted with the treated biomass, rapid metal uptake occurs. For example, when the solid biomass reaction product in the particulate state is suspended in the aqueous stream and the biomass particles become loaded with metal, the loaded particles, because of the increase in weight, settle readily and can be separated from the aqueous stream by conventional means and the metal values thereof easily recovered.

## Detailed description of the invention

Process waste waters treated in accordance with the invention may contain widely varying amounts of a heavy metal e.g., about 20 milligrams or less to about 2,000 milligrams or more per liter. To be treatable in accordance with the invention, the metal must be in cationic form. Anionic complex ions such as cyanide-metal complexes, may be decomposed by known techniques prior to treatment. Common anions such as sulfates, chlorides, nitrates, phosphates, carbonates, etc., may be present without harm and solutions can successfully be treated over the pH range of about 2 to about 11, for example, about 3 to about 11, preferably about 4 to 8, e.g., about 4 to 6. The heavy metal usually will have an atomic number greater than 20, although aluminum, atomic number 13, can be recovered from aqueous solutions.

The solid biomass product derived from *B. subtilis* cells is employed in the caustic treated condition, since greater uptake capacity for metal cations thereby results. As previously stated, the treatment preferably is effected by heating the cell biomass at 50° to 100°C or boiling, for 1 to 15 minutes with a solution preferably containing about 0.25 molar to about 1.25 molar NaOH concentration. KOH in equivalent amounts may be used. $NH_4OH$ may be employed but is not as effective. Any excess sodium or potassium hydroxide is removed by water washing. The cell biomass may be hydroxylated and/or "cleaned" by removing lipids and other organic matter which masks active sites by the caustic treatment. In any event, metal uptake by the causticized biomass is rapid, the treated biomass exhibiting high metal uptake capacity. Treatment with caustic at elevated temperatures, e.g., boiling temperatures, destroys autolytic enzymes (enzymes that the organisms possess that cause putrefaction). Biomass treated with hot or boiling sodium hydroxide solution to kill the cells and inactivate potentially putrefiable matter may be dewatered and dried prior to use. Centrifugation, filtration, etc. may be employed for dewatering. When drying is accomplished in shallow pans, a solid, hard, rock-like plate resembling slate is produced. This hard material can be crushed, ground and sized for use in a metal extraction column. When drying is accomplished in such a manner as to produce the biomass reaction product in powder form, the powder can be agglomerated for use in a metal extraction column.

Standard water treatment units may be used. For example, processing equipment comprising a mixing unit for contact between cell biomass product and metal-containing liquid may be followed by a settling unit for removing metal-containing particles from suspension with optional final filtration. This characteristic of the particulate biomass facilitates separation from the treated liquid. A continuous or batch-type ultra-filtration polymer membrane unit may be employed for mixing, sorption and filtration. Other modes for contacting metal-containing liquids with solid caustic-treated biomass include, for example, contact of the solution with the treated biomass immobilized in a matrix, such as a gel, or with a granular biomass contained in a column configuration operated as a packed or fluidized bed. During metal accumulation in caustic-treated biomass, the pH increases even though residual caustic is washed from the treated biomass. It is believed that, at equilibrium, powdered, caustic-treated biomass has greater ultimate metal-loading capacity than coarse granulated, caustic-treated biomass, this being due to the increased surface area of the powdered product. However, it is found that the caustic-treated biomass can be metal saturated more efficiently than powdered biomass reaction product if it is dried, ground, sized, for example to −35+60 mesh (0.25—0.5 mm) and then contacted with a continuous flow of metal solution at a given concentration in a packed or fluidized bed contactor. Thus, packed or fluid bed systems containing a microorganism-derived granular biomass reaction product will exhibit enhanced metal-loading capacity when compared to conventional mixing/settling systems using powdered biomass.

The metal may be recovered from the loaded biomass in a number of ways. For example, copper, silver and gold can be separated from dewatered, loaded biomass by contact with iron. The initial contact can be very small, for example, a scratch in a Teflon-coated steel pan, to initiate separation of metal from the biomass and once separation is initiated, it continues to form fan-like metal figures radiating from the initial point of contact to produce dendritic-appearing crystals of almost pure metal. Traces of iron are found near the initial contact point. High recovery of loaded metal is obtained. Precipitation of loaded metal appears to proceed on an atom-by-atom basis, at low temperature, e.g., 80°C. This observation indicates that the observed separation proceeds by galvanic action and thus indicates the invention is of particular value in extracting from solution metals which are below iron in the electromotive series; namely, cadmium, cobalt, nickel, tin, lead, antimony, arsenic, bismuth, copper, mercury, silver, palladium, platinum and gold. Other heavy metal ions can be extracted.

Metal can also be recovered pyrolytically from the loaded, dewatered biomass reaction product by fluid-bed combustion or other incineration means using the biomass as fuel.

An example will now be given:

## Example I

This example demonstrates that metal accumulation in caustic-treated biomass obtained as a biomass reaction product from *B. subtilis* is greatly enhanced as compared to that obtained with untreated *B. subtilis* biomass. Metal accumulation is also enhanced when the treated biomass is dried to a hard, solid body, ground and sized, and the biomass product in particulate form contacted countercurrently with a continuous flow of metal solution in a packed or fluidized sorption column. Results obtained in treating dilute silver and copper solutions with caustic-treated biomass as produced after boiling with 0.75M NaOH and which was then dried and ground to −60 mesh (−0.25 mm) are shown in the following Table 1:

TABLE 1

| Test | Treatment | Metal | Initial metal concentration (mg/liter) | Sorption (mg metal/g biomass, dry wt) |
|---|---|---|---|---|
| 1 | None | Ag+ | 94.4 | 11.4 |
| 2 | Boiled, 0.75M NaOH | Ag+ | 101.0 | 54.8 |
| 3 | Boiled, 0.75M NaOH, dried, sized, −60 mesh (0.25 mm) | Ag+ | 110.0 | 86.7 |
| 4 | None | $Cu^{2+}$ | 91.6 | 9.2 |
| 5 | Boiled, 0.75M NaOH | $Cu^{2+}$ | 97.4 | 35.1 |
| 6 | Boiled, 0.75M NaOH, dried, sized, −60 mesh (0.25 mm) | $Cu^{2+}$ | 97.1 | 79.2 |

Note: Tests 1 to 6 were conducted batch-wise in a complete mix reactor.

A number of other microorganisms, such as *Aspergillus flavus* and *Saccharomyces uvarum,* were given substantially the same caustic treatment as described for *Bacillus subtilis* in Table 1. The NaOH-treated microorganisms tested were examined for metal uptake and were found with respect to certain metal ions to provide improved effectiveness in recovering metal ions from solution, but in some instances, not as effective as the NaOH-treated *Bacillus subtilis*. Examples of such microorganisms are listed in Table 2 below.

TABLE 2
Microorganisms examined for metal uptake

| | |
|---|---|
| Bacteria | *Escherichia coli* |
| | *Micrococcus luteus* |
| | *Pseudomonas aeruginosa* |
| | *Thiobacillus ferrooxidans* |
| | TH3, iron oxidizing faculative thermophile |
| | *Zoogloea ramigera* |
| Fungi | *Aspergillus flavus* |
| | *Cladosporium sp.* |
| | *Neurospora crassa* |
| | *Rhizopus stolonifer* |
| Yeast | *Saccharomyces uvarum* |
| Algae | *Chlorella pyrenoidosa* |
| | *Ulothrix sp.* |

Additional tests with microorganisms have indicated that some microorganisms tended to be more selective to the recovery of certain metal ions than others. For example, a microorganism selected might be superior in metal uptake capacity as to a particular metal ion when compared to *B. subtilis*, while being less effective compared to *B. subtilis* as to another metal ion.

Thus, in carrying out the invention, the microorganism selected is that microorganism which provides enhanced metal uptake capacity as to the particular metal ion of interest following treatment of the microorganism with caustic. For example, in the treatment of a solution containing metal ions A and B and in which the metal ion of interest is B, the microorganism selected would be one which is particularly selective to the uptake of B. The remaining metal A may then be removed by a biomass reaction product produced from another microorganism more selective to A, although some A may have been taken up by the first biomass reaction product.

Comparative tests were conducted on a series of microorganisms selected from the aforementioned group consisting of bacteria, yeasts, filamentous fungi and algae. In making the comparison, *Bacillus subtilis* was used as a standard. The results indicated that certain of the microorganisms tended to be more selective to the recovery of a particular metal ion than others. As illustrative of this selectivity, the following example is given.

Example II

Different microorganisms were grown and prepared for comparison of their respective capacity for

4

metal accumulation from solutions containing lead as $Pb(NO_3)_2$ and silver as $AgNO_3$. The microorganisms were tested in the untreated and the caustic-treated state.

Biomass, which was not caustically treated, was harvested from growth medium using centrifugation. The cell paste was washed with water to remove residual medium components and the washed cell paste was oven dried and ground to yield −60 mesh particles.

Caustic-treated biomass was prepared by mixing cell paste with 3% NaOH, removing residual caustic, washing the paste, oven-drying the paste to yield a hard slate-like material, grinding and sizing the material to −60 mesh.

The oven temperature was in the neighborhood of about 80°C to 100°C.

One-half gram of each prepared biomass granule (−60 mesh (−0.25 mm) size) was added to 990 ml of an approximate 100 mg metal/liter solution shaken for 24 h at 150 rpm and 30°C. The metal loading of the granules was calculated by determining the initial and final metal concentrations in the test solution. Various conditions were used in carrying out the experiment. The biomass was either not treated with NaOH or treated using 3% NaOH. In determining the effect of pH on metal accumulation, one test was run for lead accumulation using a solution pH adjusted so that the NaOH-treated granules had a test pH near the value of that for the non-treated granules. The results are summarized in Table 3.

### TABLE 3
Comparative values for silver and lead accumulation by treated and
non-treated, granulated microorganisms

| Microorganism | Treatment | Metal | Conc. (mg/l) | pH | Metal accumulation (mg/g) | Comparative metal accumulation* |
|---|---|---|---|---|---|---|
| **Bacteria** | | | | | | |
| Bacillus | none | Ag | 94 | 5.6 | 45 | 1 |
| subtilis | NaOH | Ag | 99 | 9.6 | 65 | 1 |
| | none | Pb | 100 | 5.3 | 74 | 1 |
| | NaOH | Pb | 95 | 9.7 | 174 | 1 |
| | NaOH | Pb | 97 | 6.5 | 170 | 1 |
| | | | | | | |
| Escherichia | none | Ag | 95 | 5.5 | 54 | 1.2 |
| coli | NaOH | Ag | 93 | 10.5 | 106 | 1.6 |
| | none | Pb | 100 | 5.5 | 60 | 0.8 |
| | NaOH | Pb | 91 | 10.7 | 174 | 1.0 |
| | NaOH | Pb | 101 | 6.3 | 180 | 1.1 |
| | | | | | | |
| Pseudomonas | none | Ag | 89 | 5.4 | 36 | 0.8 |
| aeruginosa | NaOH | Ag | 90 | 8.5 | 43 | 0.7 |
| | none | Pb | 87 | 5.3 | 60 | 0.8 |
| | NaOH | Pb | 90 | 9.5 | 142 | 0.8 |
| | NaOH | Pb | 101 | 5.9 | 176 | 1.0 |
| | | | | | | |
| **Yeast** | | | | | | |
| Saccharomyces | none | Ag | 100 | 6.1 | 41 | 0.9 |
| uvarum | NaOH | Ag | 91 | 6.7 | 18 | 0.3 |
| | none | Pb | 100 | 5.6 | 86 | 1.2 |
| | NaOH | Pb | 97 | 4.5 | 109 | 0.6 |
| | | | | | | |
| **Filamentous fungi** | | | | | | |
| Neurospora | none | Ag | 93 | 6.1 | 26 | 0.6 |
| crassa | NaOH | Ag | 96 | 9.1 | 87 | 1.3 |
| | none | Pb | 81 | 4.9 | 75 | 1.0 |
| | NaOH | Pb | 80 | 9.2 | 151 | 0.9 |
| | NaOH | Pb | 101 | 4.7 | 108 | 0.6 |
| | | | | | | |
| Rhizopus | none | Ag | 93 | 5.1 | 51 | 1.1 |
| arrhizus | NaOH | Ag | 84 | 8.3 | 101 | 1.6 |
| | none | Pb | 95 | 4.2 | 118 | 1.6 |
| | NaOH | Pb | 92 | 8.6 | 171 | 1.0 |
| | | | | | | |
| Aspergillus | none | Ag | 94 | 5.5 | 24 | 0.5 |
| flavus | NaOH | Ag | 94 | 8.3 | 94 | 1.4 |
| | none | Pb | 86 | 4.7 | 34 | 0.5 |
| | NaOH | Pb | 96 | 6.6 | 179 | 1.1 |
| | | | | | | |
| **Algae** | | | | | | |
| Chlorella | none | Ag | 95 | 6.9 | 55 | 1.2 |
| vulgaris | NaOH | Ag | 96 | 8.4 | 115 | 1.8 |
| | none | Pb | 95 | 5.9 | 165 | 2.2 |
| | NaOH | Pb | 95 | 8.7 | 178 | 1.0 |
| | NaOH | Pb | 97 | 5.8 | 188 | 1.1 |
| | | | | | | |
| Chlorella | none | Ag | 95 | 6.4 | 38 | 0.8 |
| pyrenoidosa | NaOH | Ag | 99 | 9.0 | 103 | 1.6 |
| | none | Pb | 95 | 4.8 | 69 | 0.9 |
| | NaOH | Pb | 102 | 9.2 | 175 | 1.0 |
| | NaOH | Pb | 101 | 4.9 | 143 | 0.8 |

$$*\text{comparative metal accumulation} = \frac{\text{mg metal accumulated/g test microbe}}{\text{mg metal accumulated/g } B.\ subtilis}$$

The NaOH treatment enhanced accumulation of silver and lead for every microbe tested except for the yeast, *Saccharomyces uvarum,* which was selective to lead.

The enhanced lead accumulation was not solely an effect of high pH; the pH-adjusted tests using the NaOH granules indicated similar uptake regardless of pH (about 9 compared to about 6).

Comparison of the microbes tested with *B. subitilis* showed that most of the microbes exhibited improved metal uptake capacity when treated with caustic. Many of the microbes tested showed higher metal uptake capacity than *B. subitilis*. Other microorganisms, such as *Ps. aeruginosa, S. uvarum* and *A. flavus*, were not as effective, but nevertheless did show improvement with respect to certain metal ions when treated with caustic. However, *B. subtilis* is preferred in that the biomass reaction product produced therefrom is easier to handle when used to recover metal ions from aqueous solutions.

The granules produced from the *B. subitilis, E. coli* and *S. uvarum* are relatively solid but not brittle hard. In a subjective sense, the biomass reaction product might be considered soft, although they are quite stable and substantially insoluble in aqueous solutions. Biomass reaction products produced from *Ps. aeruginosa, N. crassa, R. arrhizus, A. flavus, C. vulgaris* and *C. pyrenoidosa* were subjectively softer than the biomass reaction products of the aforementioned microorganisms. However, they gave very effective results with lead and/or silver.

Not all caustic materials have the same properties of NaOH and KOH in producing granules of biomass reaction product. For example, calcium hydroxide is effective, but not as effective as NaOH. Caustic materials included within the invention are those alkaline materials which convert the microorganism into a biomass reaction product which is substantially solid and stable, capable of forming granules and which granules are substantially insoluble in the aqueous solutions being treated. The term "granules" covers particulate biomass reaction products of any particle size capable of being easily handled in a system for carrying out the process.

Granules of the substantially solid biomass reaction product are advantageous in recovering metal ions from solution in that the granules, because of their low density, can be easily suspended like a fluid bed in a column of solution in which the solution is caused to flow upwardly through a supporting column at a residence time sufficient to effect extraction of the metal ions of interest from the solution.

As the granules or particles of the biomass reaction product become loaded with the metal ions of interest, the granules settle to the bottom of the column from which they are subsequently removed.

A glass column is employed which contains the biomass granules at a specified depth. The dry weight of the granules is recorded. A solution at a specified metal concentration is pumped in the upflow direction through the column. Using this process, tests were conducted on a lead nitrate solution using a biomass reaction product produced from causticized *B. subtilis*. A plurality of one liter solutions containing lead nitrate were passed separately through the column containing 4 grams of the biomass reaction product until a total of 94 one-liter influent volumes had passed through the column. After passage of each one-liter volume, the amount of lead extracted was determined by analyzing the one-volume effluent for residual lead. When the percent recovery reaches less than about 90%, the experiment is terminated.

Following termination, the depth of the settled granules and the final weight (dry weight) are determined. The bottom one inch of granules in the column is assayed for metal content; the bottom one inch is assumed to be near saturation loading. The remaining granules in the column are blended and assayed for metal content. The results are given in Table 4. As will be noted, 99% of the metal is removed from the influent solution. This is determined by analyzing the metal content of the effluent solution.

EP 0 181 497 B1

TABLE 4
Removal of cationic lead by granules in upflow column

| Solution | Lead nitrate $(Pb(NO_3)_2)$ |
|---|---|
| pH of influent: | 5 |
| Weight of granules: | 4 g |
| Depth of granules: | 7.3 cm (2-7/8 in.) |
| Size of granules: | 0.25—0.5 mm (−35+60 mesh) |
| Superficial solution contact time: | 1.5 min. |
| Ultimate metal loading: | 1150 mg Pb/g granules (dry wt) |
| Special conditions: | Shock loadings |

| Volume processed (l) | Influent (mg/l) | Effluent (mg/l) | Effluent pH | Percent removal |
|---|---|---|---|---|
| 1.0 | 8.5 | 0.1 | 9.09 | 99 |
| 2.0 | 8.5 | 0.1 | 8.64 | 99 |
| 3.0 | 8.5 | 0.1 | 8.93 | 99+ |
| 4.0 | 8.5 | 0.1 | 8.74 | 99+ |
| 5.0 | 8.5 | 0.1 | 9.05 | 99+ |
| 6.0 | 8.5 | 0.1 | 8.71 | 99+ |
| 7.0 | 8.5 | 0.3 | 8.33 | 96 |
| 8.0 | 8.5 | 0.1 | 8.63 | 99 |
| 9.0 | 8.5 | 0.1 | 8.57 | 99 |
| 10.0 | 8.5 | 0.1 | 8.66 | 99+ |
| 11.0 | 8.5 | 0.1 | 8.87 | 99+ |
| 12.0 | 8.5 | 0.1 | 8.46 | 99+ |
| 13.0 | 8.3 | 0.1 | 7.01 | 99+ |
| 14.0 | 18.5 | 0.1 | 7.82 | 99+ |
| 15.0 | 18.5 | 0.1 | 7.57 | 99 |
| 16.0 | 18.5 | 0.1 | 7.51 | 99+ |
| 17.0 | 18.5 | 0.1 | 6.14 | 99+ |
| 18.0 | 18.5 | 0.1 | 6.40 | 99+ |
| 19.0 | 18.5 | 0.1 | 6.23 | 99 |
| 20.0 | 508.0 | 0.8 | 7.87 | 99 |
| 21.0 | 17.6 | 0.2 | 8.06 | 99 |
| 22.0 | 17.6 | 0.1 | 7.64 | 99 |
| 23.0 | 17.6 | 0.1 | 7.19 | 99 |
| 24.0 | 17.6 | 0.1 | 7.94 | 99 |
| 25.0 | 494.0 | 118.0 | 5.36 | 99 |
| 26.0 | 8.7 | 5.9 | 5.58 | 32 |
| 27.0 | 8.7 | 0.1 | 6.72 | 99+ |
| 28.0 | 9.2 | 0.1 | 6.98 | 99+ |
| 29.0 | 9.2 | — | — | — |
| 30.0 | 9.2 | — | — | — |
| 31.0 | 9.2 | 0.1 | 6.92 | 99+ |
| 32.0 | 8.9 | 0.1 | 6.64 | 99 |
| 33.0 | 8.9 | 0.1 | 6.31 | 99 |
| 34.0 | 8.9 | 0.1 | 5.82 | 99+ |
| 35.0 | 8.9 | 0.1 | 6.77 | 99+ |
| 36.0 | 8.0 | 0.1 | 6.53 | 99+ |
| 37.0 | 8.0 | 0.1 | 6.16 | 99+ |
| 38.0 | 8.0 | 0.1 | 6.32 | 99+ |
| 39.0 | 7.3 | 0.1 | 6.06 | 99 |
| 40.0 | 7.3 | 0.1 | 6.35 | 99+ |
| 41.0 | 7.3 | 0.1 | 6.56 | 99+ |
| 42.0 | 7.3 | 0.1 | 6.42 | 99+ |
| 43.0 | 7.3 | 0.1 | 6.44 | 99+ |
| 44.0 | 7.3 | 0.1 | 6.14 | 99+ |
| 45.0 | 7.3 | 0.2 | 6.49 | 99 |
| 46.0 | 8.1 | 0.8 | 6.47 | 95 |
| 47.0 | 8.1 | 0.1 | 6.35 | 99+ |
| 48.0 | 8.2 | 0.1 | 6.51 | 99 |
| 49.0 | 8.2 | 0.2 | 6.51 | 99 |

8

TABLE 4 (cont.)

| Volume processed (l) | Influent (mg/l) | Effluent (mg/l) | Effluent pH | Percent removal |
|---|---|---|---|---|
| 50.0 | 8.2 | 0.1 | 6.28 | 99 |
| 51.0 | 8.2 | 0.1 | 5.94 | 99 |
| 52.0 | 8.2 | 0.1 | 6.11 | 99 |
| 53.0 | 20.4 | 0.1 | 6.51 | 99 |
| 55.0 | 20.4 | 0.2 | 6.25 | 99 |
| 57.0 | 20.1 | 0.1 | 6.32 | 99+ |
| 59.0 | 20.1 | 0.1 | 6.90 | 99 |
| 61.0 | 20.1 | 0.1 | 6.66 | 99+ |
| 63.0 | 16.8 | 0.1 | 6.50 | 99+ |
| 65.0 | 16.8 | 0.1 | 6.17 | 99+ |
| 67.0 | 16.8 | 0.1 | 6.24 | 99 |
| 69.0 | 19.0 | 0.1 | 5.63 | 99+ |
| 71.0 | 19.0 | 0.1 | 5.67 | 99 |
| 73.0 | 18.8 | 0.1 | 6.42 | 99 |
| 75.0 | 18.8 | 0.1 | 6.53 | 99+ |
| 77.0 | 18.8 | 0.1 | 6.25 | 99+ |
| 79.0 | 18.8 | 0.2 | 6.09 | 99 |
| 81.0 | 19.8 | 0.4 | — | 98 |
| 83.0 | 19.8 | 0.3 | 5.96 | 98 |
| 85.0 | 19.8 | 0.1 | 5.98 | 99 |
| 87.0 | 19.2 | 0.2 | 6.03 | 99 |
| 89.0 | 19.2 | 0.8 | — | 96 |
| 91.0 | 18.9 | 0.8 | 5.90 | 96 |
| 93.0 | 18.9 | 4.6 | 5.53 | 76 |
| 94.0 | 18.9 | 1.5 | 5.44 | 92 |

The solutions tested were dilute, except for two solutions (volumes 20 and 25) which contained shock loadings of 508 and 494 mg/l of lead, respectively. Each pass-through of solution from volumes 1 to 87 showed substantial removal of lead of about 99% for each volume. This test illustrates the very high metal uptake capacity of the 4 grams of biomass reaction product, despite the shock loading of volumes 20 and 25. Substantially all of the lead was removed from the solution by the granules.

Additional tests were conducted on copper sulfate solution. These tests are reported in Tables 5 and 6. The solutions treated in Table 5 had a fairly high influent loading of copper of about 100 mg/l; whereas, in Table 6, the influent loading of copper was much lower and ranged from about 8.5 to 9.5 mg/l of copper. Both tests showed a fairly high metal uptake capacity of the biomass reaction product.

TABLE 5

Removal of cationic copper by granules in upflow column
(influent=100 mg/Cu/l)

| | |
|---|---|
| Solution: | Copper sulfate ($CuSO_4$) |
| pH of influent: | 5 |
| Weight of granules: | 5.75 g |
| Depth of granules: | 9.53 cm (3.75 in.) |
| Size of granules: | 0.25—0.5 mm (−35+60 mesh) |
| Superficial solution contact time: | 2.3 min. |
| Ultimate metal loading: | 150.7 mg Cu/g granules (dry wt) |
| Special conditions: | none |

| Volume processed (l) | Influent (mg/l) | Effluent (mg/l) | Effluent pH | Percent removal |
|---|---|---|---|---|
| 1 | 100 | 0.1 | 7.3 | 99+ |
| 2 | 100 | 0.3 | 6.9 | 99 |
| 3 | 100 | 2.5 | 6.2 | 97 |
| 5 | 100 | 6.1 | 6.2 | 94 |
| 7 | 100 | 24.8 | 5.6 | 75 |
| 9 | 100 | 49.7 | 5.0 | 50 |

# EP 0 181 497 B1

TABLE 6
Removal of cationic copper by granules in upflow column
(influent=10 mg/Cu/l)

| Solution: | Copper sulfate (CuSO₄) |
|---|---|
| pH of influent: | 5.41 |
| Weight of granules: | 6.5 g |
| Depth of granules: | 10.57 cm (4.16 in.) |
| Size of granules: | 0.25—0.5 mm (−35+60 mesh) |
| Superficial solution contact time: | 2.3 min. |
| Ultimate metal loading: | 140 mg Cu/g granules (dry wt) |
| Special conditions: | none |

| Volume processed (l) | Influent (mg/l) | Effluent (mg/l) | Effluent pH | Percent removal |
|---|---|---|---|---|
| 1 | 8.5 | 0.1 | 9.66 | 99 |
| 2 | 8.5 | 0.2 | 7.94 | 98 |
| 3 | 8.5 | 0.3 | 6.94 | 96 |
| 5 | 9.1 | 0.1 | 6.93 | 99 |
| 7 | 9.1 | 0.1 | 6.83 | 99+ |
| 9 | 9.2 | 0.1 | 7.31 | 99+ |
| 11 | 9.2 | 0.1 | 6.85 | 99+ |
| 13 | 9.2 | 0.1 | 6.42 | 99 |
| 15 | 9.3 | 0.1 | 6.85 | 99 |
| 17 | 9.3 | 0.2 | 7.05 | 98 |
| 19 | 9.6 | 0.1 | 6.54 | 99+ |
| 21 | 9.6 | 0.1 | 6.53 | 99+ |
| 23 | 9.5 | 0.4 | — | 96 |
| 25 | 9.5 | 0.4 | 6.56 | 96 |
| 27 | 9.5 | 0.1 | 7.06 | 99+ |
| 29 | 8.5 | 0.1 | 7.45 | 99+ |
| 31 | 8.5 | 0.1 | 7.68 | 99+ |
| 33 | 8.5 | 0.1 | 7.05 | 99+ |
| 35 | 8.5 | 0.1 | 6.75 | 99+ |
| 37 | 9.0 | 0.1 | 6.77 | 99 |
| 39 | 9.0 | 0.1 | 6.87 | 99 |
| 41 | 9.0 | 0.1 | 6.89 | 99+ |
| 43 | 9.0 | 0.1 | 6.95 | 99+ |
| 45 | 9.0 | 0.1 | 6.74 | 99 |
| 47 | 9.4 | 0.1 | 6.81 | 99 |
| 49 | 9.4 | 0.2 | 6.48 | 98 |
| 51 | 9.4 | 0.1 | 6.64 | 99 |
| 53 | 9.4 | 0.2 | 6.31 | 98 |
| 55 | 9.0 | 0.8 | 5.85 | 91 |
| 57 | 9.0 | 1.1 | 5.98 | 88 |
| 58 | 9.0 | 1.2 | 5.96 | 87 |

As will be apparent from the tests summarized in Tables 4 to 6, a biomass reaction product may exhibit different metal uptake properties for different metal ions. Likewise, as will be apparent from Table 3, different biomass reaction products exhibit different selectivity for different metal ions.

Despite such differences, the biomass reaction products described herein have great utility. For example, where it is desired to cleanup wastewater containing toxic metal ions, at least two of which are selective to different biomass products, a mixture of two or more biomass products may be employed to remove the toxic elements.

With regard to the caustic reagent used to carry out the invention, it has been found that a strong detergent material sold under the trademark "Alconox" appears to act similarly to sodium hydroxide in treating *B. subtilis*. This detergent is a long chain alkyl sulfonate which is a wetting agent. It is biodegradable. It contains about 7.3% phosphorus by weight as trisodium phosphate. The pH of the detergent is 9 to 9.5.

Attempts have been made to explain the reason for the selective behavior of caustic-treated microorganisms. While we do not wish to be held to any particular theory, it has been stated that the cell walls of *Bacillus subtilis* (a Gram-positive bacterium) are considered to be polymeric in nature including strands of mucopeptides (peptidoglycans), teichoic acids or teichuronic acids distributed and attached

10

along their length. The long polymers comprising the mucopeptides are composed of three kinds of building blocks; namely, N-acetylglucosamine (AGA), N-acetylmuramic acid (AMA), and a peptide consisting of four or five amino acids; i.e., L-alanine, D-alanine, D-glutamic acid and either lysine or diaminopimelic acid. A substantial proportion, e.g., about 35% of the mucopeptide strands are cross-linked together through transpeptide (D-alanyl-(L)-meso-diaminopimelyl) bonds. The polymeric structure possesses chemically reactive groups including oxygen, nitrogen, sulfur and phosphorus which carry lone electron pairs that bind metals. Such atoms are effective as electron donors for metal ions. The effects of sodium hydroxide upon such materials are not readily evident although, inter alia, it is believed sodium hydroxide removes lipids and other organic impurities that mask reactive sites. Caustic-treated cell wall material from other Gram-positive bacilli having mucopeptide strands cross-linked with peptide bonds and reacting similarly to caustic treatment may also be employed in accordance with the invention.

The Gram-negative bacterium, *E. coli*, is also particularly useful as a starting material, in carrying out the invention.

Conversion of the caustic-treated biomass derived from *B. subtilis* cells into particles by drying to a hard or solid body and grinding facilitates handling and promotes treatment of metal-containing aqueous streams to recover the metal content thereof by continuous counter-current decantation, by pulsed bed, fluid bed and other technologies in which a column of liquid undergoing treatment can be employed. Thus the granular biomass may be configured in packed columns suitable for upflow or downflow operation or it may be employed in an unrestrained, upflow column allowing fluidization thereof.

The biomass may be ground to provide a variety of particle sizes and employed in an upflow column through which a metal-containing aqueous solution to be treated is passed at such a rate that stratification or classification of the biomass particles results, with coarser particles in the lower portion of the bed and finer, more active particles in the upper portion of the bed. In this way, the coarser particles contact fresh feed while the finer particles contact a partially metal depleted stream after the bulk of the metal cations have been removed by the larger particles. This action promotes high loading of the particles and high recovery of metal from the incoming stream.

In another embodiment of the process, the column of biomass is operated as a pulsed bed with the aqueous solution to be treated moving upwardly in the column and fresh biomass is introduced portionwise at a point toward the top of the column while metal-laden biomass is removed from a point toward the bottom of the column. The volumes of the portions of fresh and metal-laden biomass may be substantially the same, and the metal-laden biomass portions may be removed and the fresh portions introduced at substantially the same time.

In summary, the invention comprises various embodiments.

One embodiment is directed to a process for enhancing the metal uptake properties of *Bacillus subtilis* from aqueous solutions containing metal cations in which cells of the bacterium, *Bacillus subtilis*, are treated with a caustic solution to form a causticized material consisting essentially of a biomass reaction product. In another embodiment, the biomass reaction product is recovered and dried to form a relatively solid, stable product. The biomass reaction product in the particulate state exhibits substantially enhanced metal uptake capacity as compared to the metal uptake capacity of the *Bacillus subtilis* in the untreated state. A preferred caustic solution is one selected from the group consisting of NaOH and KOH.

As stated herein, the caustic treatment may be carried out at temperatures ranging up to boiling and preferably over a temperature range of about 50°C to about 100°C.

The process is applicable to a relatively broad range of microorganisms provided they are characterized by cell wall structures. Thus, the process is applicable to cells of bacilli, such as Gram-positive bacteria (e.g., *B. subtilis*) and Gram-negative bacteria (e.g., *E. coli*). Examples of microorganisms are bacteria, yeast, fungi and algae, so long as the microorganism is characterized by a cell wall structure and is selective to uptake of metal ions, and which when treated with a caustic solution produces a relatively solid, stable biomass reaction product having substantially enhanced metal uptake capacity for metal ions compared to the uptake capacity before treatment.

The biomass reaction product of the invention consists essentially of a causticized non-living microorganism.

A particular advantage of the invention is that a microorganism-derived biomass reaction product, in the form of a solid, stable particulate material, can be used as a fluid bed in an aqueous solution containing metal cations with the solution flowing countercurrently through the bed. As the particles of the bed become loaded, they segregate to a lower part of the bed from which they can be removed for the subsequent recovery of metal therefrom.

## Claims

1. A non-living biomass reaction product having the ability to take up metal cations from aqueous solutions, characterised in that it comprises a microorganism having a cell wall structure treated with caustic alkali solution whereby its metal uptake properties have been enhanced.

2. A biomass reaction product according to claim 1 in dried, relatively solid and stable form.

3. A biomass reaction product according to claim 2 in ground, particulate form.

4. A biomass reaction product according to claim 1 or claim 2 wherein said microorganism is a bacterium.

5. A biomass reaction product according to claim 4 wherein said bacterium is a Gram-positive bacterium having mucopeptide strands cross-linked with transpeptide bonds.

6. A biomass reaction product according to claim 4 wherein said bacterium is a *Bacillus* species.

7. A biomass reaction product according to claim 6 wherein said bacterium is *Bacillus subtilis*.

8. A process in which a microorganism having a cell wall structure and the ability to take up metal cations from aqueous solutions is treated with caustic alkali solution at a pH above 9 and the treated microorganism is recovered and dried to form a relatively solid, stable biomass reaction product having enhanced metal uptake properties compared with the microorganism treated.

9. A process according to claim 8 wherein said microorganism is a bacterium.

10. A process according to claim 9 wherein said microorganism is a Gram-positive bacterium having mucopeptide strands cross-linked with transpeptide bonds.

11. A process according to claim 9 wherein said microorganism is a *Bacillus* species.

12. A process according to claim 11 wherein said microorganism is *Bacillus subtilis*.

13. A process according to any one of claims 8 to 12 wherein the caustic alkali is NaOH or KOH and the treatment is carried out at a temperature in the range from about 50°C to about 100°C.

14. A process in which a biomass reaction product according to any one of claims 1 to 7 or made by the process of any one of claims 8 to 13 is used to remove metal cations from solution.

15. A process according to claim 14 wherein the cations are heavy metal cations and the solution is contacted with a biomass comprising polymeric cell wall material derived from a Gram-positive bacterium having mucopeptide strands cross-linked with transpeptide bonds which have been treated with a base from the group consisting of sodium hydroxide and potassium hydroxide to sorb said cation into said biomass and the resulting metal-containing biomass is thereafter separated from said solution.

16. A process according to claim 15 in which the biomass is derived from *Bacillus subtilis*.

17. A process according to claim 16 wherein said biomass is treated with a hot, dilute caustic solution prior to said contact.

18. A process according to claim 17 wherein said metal-containing solution is a waste water solution containing up to about two grams per liter of said metal.

19. A process according to any one of claims 16 to 18 wherein said heavy metal is silver, gold or a platinum group metal.

20. A process according to any one of claims 16 to 19 wherein the metal-containing biomass is treated galvanically to recover said metal.

21. A process according to any one of claims 16 to 19 wherein said metal-containing biomass is treated pyrolytically to recover said metal.

22. A process according to any one of claims 16 to 21 wherein said aqueous solution is a waste water stream, said treated biomass is added to and mixed with said stream and said metal-containing biomass is removed from said stream by flocculation and settling.

23. A process according to claim 14 wherein the cations are heavy metal cations and the solution is passed through a column of granular biomass comprising cells of the bacterium *Bacillus subtilis* which have been treated with a base from the group consisting of sodium hydroxide and potassium hydroxide, dried to a hard, grindable body and ground to granular form.

24. A process according to claim 23 wherein said granular biomass is configured in a packed column suitable for upflow operation.

25. A process according to claim 23 wherein said granular biomass is configured in a packed column suitable for downflow operation.

26. A process according to claim 23 wherein said granular biomass is employed in an unrestrained, upflow column allowing fluidization of said granular biomass.

27. A process according to claim 23 wherein said grindable body of treated biomass is ground to provide a variety of particle sizes, said ground treated biomass is employed in an upflow column wherein said aqueous solution is passed at a rate causing stratification of said biomass with larger biomass particles in the lower portion of said column and finer biomass particles in the upper portions of said column whereby the smaller and more active biomass particles contact said solution after the bulk of said cation has been removed therefrom by said larger particles.

28. A process according to claim 23 wherein said column is operated as a pulsed bed of said biomass, with said aqueous solution moving upwardly in said column, with fresh treated biomass introduced portionwise at a point toward the top of said column while metal-laden biomass is removed from a point toward the bottom of said column.

29. A process according to claim 28 wherein the respective volumes of said fresh biomass portions and said metal-laden biomass portions are substantially the same.

30. A process according to claim 28 or 29 wherein said metal-laden biomass portions are removed and said fresh biomass potions are introduced at substantially the same time.

**Patentansprüche**

1. Reaktionsprodukt aus einer nicht lebenden Biomasse mit der Fähigkeit zur Aufnahme metallischer Kationen aus wässerigen Lösungen, gekennzeichnet durch einen Gehalt an einem Mikroorganismus mit einer durch Behandlung mit ätzalkalischen Lösungen in den Metallaufnahme-Eigenschaften verbesserten Zellwandstruktur.

2. Biomassen-Reaktionsprodukt gemäß Anspruch 1, das in getrockneter, relativ fester und stabiler Form vorliegt.

3. Biomassen-Reaktionsprodukt gemäß Anspruch 2, das in vermahlener Partikelform vorliegt.

4. Biomassen-Reaktionsprodukt gemäß Anspruch 1 oder 2, bei dem der Mikroorganismus ein Bakterium ist.

5. Biomassen-Reaktionsprodukt gemäß Anspruch 4, bei dem das Bakterium ein grampositives Bakterium mit durch transpeptide Bindungen vernetzten mucopeptiden Strängen ist.

6. Biomassen-Reaktionsprodukt gemäß Anspruch 4, bei dem das Bakterium ein solches der Bacillus-Art ist.

7. Biomassen-Reaktionsprodukt gemäß Anspruch 6, bei dem das Bakterium Bacillus subtilis ist.

8. Verfahren, bei dem ein Mikroorganismus mit einer Zellwandstruktur und der Fähigkeit zur Aufnahme von Metallkationen aus wässerigen Lösungen mit einer ätzalkalischen Lösung bei einem pH über 9 behandelt wird und der behandelte Mikroorganismus wiedergewonnen und getrocknet wird zur Gewinnung eines relativ festen stabilen Biomassen-Reaktionsprodukts das im Vergleich zu dem unbehandelten Mikroorganismus verbesserte Metallaufnahme-Eigenschaften besitzt.

9. Verfahren gemäß Anspruch 8, bei dem der Mikroorganismus ein Bakterium ist.

10. Verfahren gemäß Anspruch 9, bei dem der Mikroorganismus ein grampositives Bakterium mit durch transpeptide Bindungen vernetzten mucopeptiden Strängen ist.

11. Verfahren gemäß Anspruch 9, bei dem der Mikroorganismus ein solcher der Bacillus-Art ist.

12. Verfahren gemäß Anspruch 11, bei dem der Mikroorganismus Bacillus subtilis ist.

13. Verfahren gemäß einem jeden der Ansprüche 8 bis 12, bei dem das Ätzalkali NaOH oder KOH ist und die Behandlung bei einer Temperatur im Bereich von etwa 50°C bis etwa 100°C durchgeführt wird.

14. Verfahren bei dem das Biomassen-Reaktionsprodukt gemäß einem der Ansprüche 1 bis 7 oder hergestellt gemäß einem der Ansprüche 8 bis 13 zur Entfernung von Metallkationen aus Lösungen verwendet wird.

15. Verfahren gemäß Anspruch 14, bei dem die Kationen Schwermetallkationen sind und die Lösungen mit einer Biomasse mit polymerem Zellwand-Material aus einem grampositiven Bakterium mit durch transpeptide Bindungen vernetzten mucopeptiden Strängen, das mit einer Base ausgewählt aus der von Natriumhydroxid und Kaliumhydroxid gebildeten Gruppe behandelt worden ist, zur Sorption der Kationen in Berührung gebracht werden und die resultierende metallhaltige Biomasse aus den Lösungen abgetrennt wird.

16. Verfahren gemäß Anspruch 15, bei dem die Biomasse von Bacillus subtilis abgeleitet ist.

17. Verfahren gemäß Anspruch 16, bei dem die Biomasse vor dem Inberührungbringen mit einer heißen, verdünnten ätzalkalischen Lösung behandelt worden ist.

18. Verfahren gemäß Anspruch 17, bei dem die metallhaltige Lösung eine Abwasserlösung mit einem Gehalt an dem Metall von bis zu etwa 2 g pro Liter ist.

19. Verfahren gemäß einem jeden der Ansprüche 16 bis 18, bei dem das Schwermetall Silber, Gold oder ein solches aus der Platin-Gruppe ist.

20. Verfahren gemäß einem jeden der Ansprüche 16 bis 19, bei dem die metallhaltige Biomasse zur Rückgewinnung des Metalls galvanisch behandelt wird.

21. Verfahren gemäß einem jeden der Ansprüche 16 bis 19, bei dem die metallhaltige Biomasse zur Rückgewinnung des Metalls pyrolytisch behandelt wird.

22. Verfahren gemäß einem jeden der Ansprüche 16 bis 21, bei dem die wässerige Lösung ein Abwasserstrom ist, die behandelte Biomasse diesem zugegeben und mit demselben vermischt wird und die metallhaltige Biomasse aus dem Strom durch Flockung und Absetzen entfernt wird.

23. Verfahren gemäß Anspruch 14, bei dem die Kationen Schwermetallkationen sind und die Lösung durch eine Kolonne einer granulierten Biomasse aus Zellen des Bakteriums Bacillus subtilis geführt wird, die mit einer Base aus der von Natriumhydroxid und Kaliumhydroxid gebildeten Gruppe behandelt und zu einem harten vermahlbaren Körper getrocknet sowie in Granulatform vermahlen worden sind.

24. Verfahren gemäß Anspruch 23, bei dem die granulierte Biomasse in einer gepackten, für den Betrieb im Aufwärtsfluß geeigneten Kolonne angeordnet ist.

25. Verfahren gemäß Anspruch 23, bei dem die granulierte Biomasse in einer gepackten, für den Betrieb im Abwärtsfluß geeigneten Kolonne angeordnet ist.

26. Verfahren gemäß Anspruch 23, bei dem die granulierte Biomasse in einer ungedämmten aufwärtsfließenden Kolonne, die eine Fluidisierung der Biomasse erlaubt, verwendet wird.

27. Verfahren gemäß Anspruch 23, bei dem der vermahlbare Körper aus der behandelten Biomasse unter Bildung verschiedener Teilchengrößen vermahlen wird, die vermahlene behandelte Biomasse in einer Kolonne mit Aufwärtsfluß verwendet wird, wobei die wässerige Lösung in einer solchen Menge durchgeführt wird, daß die größeren Teilchen der Biomasse im unteren Teil der Kolonne und die feineren Teilchen der Biomasse in den oberen Abschnitten der Kolonne geschichtet sind und die kleineren, aktiveren Teilchen der Biomasse mit der Lösung in Berührung kommen, nachdem der Großteil der Kationen daraus durch die größeren Teilchen entfernt worden ist.

28. Verfahren gemäß Anspruch 23, bei dem die Kolonne als ein Stoßbett der Biomasse betrieben wird, wobei die wässerige Lösung sich in der Kolonne nach oben bewegt und die frische, behandelte Biomasse portionweise an einem Punkt in Richtung auf den Kopf der Kolonne eingebracht wird sowie die metallhaltige Biomasse an einem Punkt in Richtung auf den Boden der Kolonne zur Entfernung gelangt.

29. Verfahren gemäß Anspruch 28, bei dem die jeweiligen Volumen der frischen Biomassenanteile und der metallhaltigen Biomassenanteile im wesentlichen gleich sind.

30. Verfahren gemäß Anspruch 28 oder 29, bei dem die metallhaltigen Biomassenanteile zur im wesentlichen gleichen Zeit entfernt werden wie die frischen Biomassenanteile zugeführt werden.

**Revendications**

1. Un produit de réaction de biomasse non vivant ayant la capacité d'extraire des cations métalliques des solutions aqueuses, caractérisé en ce qu'il comprend un microorganisme ayant une structure à paroi cellulaire traité par une solution d'alcali caustique en vue d'améliorer ses propriétés d'extraction des métaux.

2. Un produit de réaction de biomasse selon la revendication 1, sous une forme séchée, relativement solide et stable.

3. Un produit de réaction de biomasse selon la revendication 2, sous une forme broyée, particulaire.

4. Un produit de réaction de biomasse selon la revendication 1 ou 2, selon laquelle ledit microorganisme est une bactérie.

5. Un produit de réaction de biomasse selon la revendication 4, selon laquelle ladite bactérie est une bactérie Gram positif ayant des chaînes de mucopeptide réticulées par des liaisons transpeptides.

6. Un produit de réaction de biomasse selon la revendication 4, selon laquelle ladite bactérie est une espèce de *Bacillus*.

7. Un produit de réaction de biomasse selon la revendication 6, selon laquelle ladite bactérie est le *Bacillus subtilis*.

8. Un procédé selon lequel un microorganisme ayant une structure à paroi cellulaire et la capacité d'extraire les cations métalliques des solutions aqueuses est traité par une solution d'alcali caustique à un pH supérieur à 9 et le microorganisme traité est récupéré et séché pour former un produit de réaction de biomasse stable relativement solide ayant des propriétés d'extraction de métaux améliorées comparativement au microorganisme non traité.

9. Un procédé selon la revendication 8, selon laquelle ledit microorganisme est une bactérie.

10. Un procédé selon la revendication 9, selon laquelle ledit microorganisme est une bactérie Gram positif ayant des chaînes mucopeptides réticulées par des liaisons transpeptides.

11. Un procédé selon la revendication 9, selon laquelle ledit microorganisme est un espèce de *Bacillus*.

12. Un procédé selon la revendication 11, selon laquelle ledit microorganisme est le *Bacillus subtilis*.

13. Un procédé selon l'une quelconque des revendications 8 à 12, selon laquelle l'alcali caustique est le NaOH ou le KOH et le traitement est conduit à une température comprise dans l'intervalle d'environ 50°C à environ 100°C.

14. Un procédé selon lequel un produit de réaction de biomasse selon l'une quelconque des revendications 1 à 7 ou obtenu par le procédé selon l'une quelconque des revendications 8 à 13 est utilisé pour séparer les cations métalliques des solutions.

15. Un procédé selon la revendication 14, selon laquelle les cations sont des cations de métaux lourds et la solution est mise en contact avec une biomasse comprenant une matière à paroi cellulaire polymère dérivée d'une bactérie Gram positive ayant des chaînes mucopeptides réticulées par des liaisons transpeptides qui a été traitée par une base choisie dans le groupe comprenant l'hydroxyde de sodium et l'hydroxyde de potassium pour sorber ledit cation dans ladite biomasse et la biomasse contenant les métaux résultante est ensuite séparée de ladite solution.

16. Un procédé selon la revendication 15, selon laquelle la biomasse est dérivée du *Bacillus subtilis*.

17. Un procédé selon la revendication 16, selon laquelle ladite biomasse est traitée par une solution de caustique diluée chaude avant ledit contact.

18. Un procédé selon la revendication 17, selon laquelle ladite solution contenant un métal est une solution d'eaux résiduaires contenant jusqu'à environ 2 g/l dudit métal.

19. Un procédé selon l'une quelconque des revendications 16 à 18, selon laquelle ledit métal lourd est l'argent, l'or ou un métal du groupe du platine.

20. Un procédé selon l'une quelconque des revendications 16 à 19, selon laquelle la biomasse contenant le métal est traitée galvaniquement pour récupérer ledit métal.

21. Un procédé selon l'une quelconque des revendications 16 à 19, selon laquelle ladite biomasse contenant un métal est traitée pyrolytiquement pour récupérer ledit métal.

22. Un procédé selon l'une quelconque des revendications 16 à 21, selon laquelle ladite solution aqueuse est un courant d'eaux résiduaires, ladite biomasse traitée est ajoutée et mélangée audit courant et ladite biomasse contenant un métal est séparée dudit courant par floculation et décantation.

23. Un procédé selon la revendication 14, selon laquelle les cations sont des cations de métaux lourds et la solution est envoyée à travers une colonne de biomasse granulaire comprenant des cellules de la bactérie *Bacillus subtilis* qui ont été traitées par une base dans le groupe comprenant l'hydroxyde de sodium et l'hydroxyde de potassium, séchées en un corps dur, broyable, et broyées sous une forme granulaire.

24. Un procédé selon la revendication 23, selon laquelle ladite biomasse granulaire est agencée en une colonne tassée appropriée pour le fonctionnement à courant ascendant.

25. Un procédé selon la revendication 23, selon laquelle ladite biomasse granulaire est agencée en une colonne tassée appropriée pour le fonctionnement à courant descendant.

26. Un procédé selon la revendication 23, selon laquelle ladite biomasse granulaire est employée dans une colonne à courant ascendant non rétreinte permettant la fluidisation de ladite biomasse granulaire.

27. Un procédé selon la revendication 23, selon laquelle ledit corps broyable de biomasse traitée est broyé pour donner diverses tailles de particules, la biomasse traitée broyée est employée dans une colonne à courant ascendant, selon lequel ladite solution aqueuse circule à un débit approprié pour provoquer la stratification de ladite biomasse avec des particules de biomasse plus grosses dans la portion inférieure de ladite colonne et des particules de biomasse plus fines dans les portions supérieures de ladite colonne, de sorte que les particules de biomasse plus petites et plus actives sont en contact avec la solution après que la plus grande partie dudit cation a été séparée par lesdites particules plus grosses.

28. Un procédé selon la revendication 23, selon laquelle ladite colonne fonctionne en lit pulsé de ladite biomasse, avec ladite solution aqueuse se déplaçant en courant ascendant dans ladite colonne avec la biomasse traitée fraîche introduite par portions en un point dans la direction du sommet de ladite colonne tandis que la biomasse chargée de métal est séparée à partir d'un point dans la direction du fond de ladite colonne.

29. Un procédé selon la revendication 28, selon laquelle les volumes respectifs desdites portions de biomasse fraîche et desdites portions de biomasse chargées de métal sont substantiellement les mêmes.

30. Un procédé selon la revendication 28 ou 29, selon laquelle lesdites portions de biomasse chargées de métal sont séparées et lesdites portions de biomasse fraîches sont introduites substantiellement en même temps.